# EUROPÄISCHE PATENTSCHRIFT

(11) **EP 1 012 143 B1**
(45) Veröffentlichungstag und Bekanntmachung des Hinweises auf die Patenterteilung: **17.12.2003**
(21) Anmeldenummer: 98940248.2
(22) Anmeldetag: 06.08.1998
(51) Int. Cl.: C07D 231/06

(54) **VERFAHREN ZUR HERSTELLUNG VON 1-PHENYLPYRAZOLIN-3-CARBONSÄURE-DERIVATEN**
METHOD FOR PREPARING 1-PHENYLPYRRAZOLIN-3-CARBOXYLIC ACID DERIVATIVES
PROCEDE POUR LA PREPARATION DE DERIVES D'ACIDE 1-PHENYLPYRRAZOLIN-3-CARBOXYLIQUE

(30) Priorität: 09.09.1997 DE 19739489
(43) Veröffentlichungstag der Anmeldung: 28.06.2000
(73) Patentinhaber: Bayer CropScience GmbH, 65929 Frankfurt/Main (DE)
(72) Erfinder: SCHLEGEL, Günter, Tokyo 158 (JP)
(86) Internationale Anmeldenummer: EP9804895
(87) Internationale Veröffentlichungsnummer: WO99012909

(56) Entgegenhaltungen:
- EP-A- 0 409 118
- WO-A-88/06583
- WO-A-91/07874

## Beschreibung

Die vorliegende Erfindung betrifft ein Verfahren zur Herstellung von 1-Phenylpyrazolin-3-carbonsäure-Derivaten durch basenkatalysierte Cycloaddition von 2-Phenylhydrazonverbindungen mit substituierten Olefinen in einem Zweiphasen-System.

Pyrazoline können - wie auch andere fünfgliedrige heterocyclische Verbindungen - beispielsweise durch [3+2]-Cycloaddition ungesättigter Verbindungen mit 1,3-dipolaren Molekülen hergestellt werden, siehe z.B. Jerry March, "Advanced Organic Chemistry", 3rd Ed., John Wiley & Sons, N.Y. 1985, Seiten 743-745 und dort zitierte Literatur. Da zahlreiche 1,3-dipolare Moleküle oftmals nicht lagerstabil sind, werden zweckmäßigerweise an ihrer Stelle solche Verbindungen eingesetzt, die in situ unter Baseneinfluß zu 1,3-dipolaren Molekülen reagieren. Diese Reaktionen werden üblicherweise in Gegenwart einer Base mit oder ohne Lösungsmittel durchgeführt.

Aus WO 91/07874 sind einige 1-Phenylpyrazolin-3-carbonsäureester als Verbindungen bekannt geworden, die die Nutzpflanzen in land- und forstwirtschaftlichen Kulturen vor unerwünschten Schäden bei der Einwirkung von Herbiziden schützen. Die dort beschriebene Synthese von 1-Phenylpyrazolin-3-carbonsäureestern wird so durchgeführt, daß 2-Phenylhydrazone von Halogenglyoxalsäureestern unter Basenkatalyse mit substituierten Olefinen umgesetzt werden. Diese Verfahren ist jedoch mit einigen Nachteilen verbunden:
a) nicht ausreichende Reaktionsausbeute,
b) ungenügende Reinheit des Produkts,
c) hoher Verbrauch der als Base eingesetzten tertiären Amine,
d) aufwendige Entsorgung des bei der Reaktion entstandenen Salzes aus Halogenwasserstoff und tertiärem Amin,
e) hoher im Produkt verbleibender Restanteil nicht abreagierten und toxikologisch bedenklichen 2-Phenylhydrazons,
f) Gefahr der Polymerisation der Olefine unter den dort herrschenden basischen Bedingungen.

Aufgabe der vorliegenden Erfindung ist daher die Bereitstellung eines Verfahrens, das eine kostengünstige Herstellung von 1-Phenylpyrazolin-3-carbonsäure-Derivaten in hoher Reinheit erlaubt.

Die Lösung der Aufgabe geht aus von dem bekannten Verfahren zur Herstellung von 1-Phenylpyrazolin-3-carbonsäure-Derivaten der allgemeinen Formel III durch basenkatalysierte Reaktion von Hydrazonen der allgemeinen Formel I mit Olefinen der allgemeinen Formel II 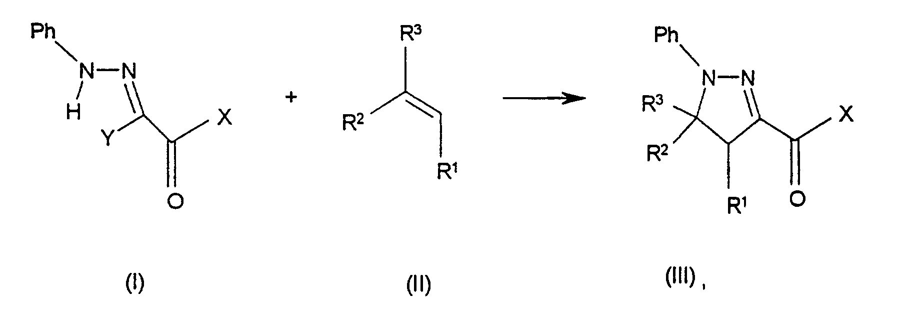 worin
- Ph: gegebenenfalls substituiertes Phenyl,
- R¹: Wasserstoff oder Alkyl,
- R² und R³: unabhängig voneinander Wasserstoff, Halogen, Cyano, einen gegebenenfalls substituierten organischen Rest, oder R² und R³ mit dem sie tragenden Kohlenstoffatom einen gesättigten oder teilgesättigten 5- oder 6-atomigen Ring bildend,
- X: Amino, Hydroxy, Alkoxy, Cycloalkoxy, Alkylamino, Dialkylamino, Alkyloxyalkyloxy, Trialkylsilyloxy oder Trialkylsilylmethyloxy und
- Y: Chlor oder Brom
bedeuten.

Das erfindungsgemäße Verfahren ist dann dadurch gekennzeichnet, daß die Reaktion in einem Zweiphasen-System in Anwesenheit eines sterisch gehinderten Amins und gegebenenfalls einer weiteren Base durchgeführt wird.

Für das vorliegende Verfahren ist der Einsatz oben genannter Verbindungen bevorzugt, in denen Ph, R¹ und Y wie vorstehend definiert sind und
- R² und R³: unabhängig voneinander Wasserstoff, Halogen, Cyano, C₁-C₆-Alkyl, C₃-C₆-Cycloalkyl, C₂-C₆-Alkenyl, C₂-C₆-Alkinyl, Halogen-C₁-C₆-alkyl, C₁-C₆-Alkoxy-C₁-C₆-alkyl, C₁-C₆-Alkylcarbonyl, C₁-C₆-Alkoxycarbonyl, C₁-C₆-Alkylaminocarbonyl, Di-(C₁-C₆-alkyl)-aminocarbonyl, unsubstituiertes oder durch ein oder mehrere gleiche oder verschiedene Reste aus der Gruppe Amino, Carboxyl, Cyano, Formyl, Halogen, Hydroxy, Nitro, C₁-C₄-Alkyl, C₁-C₄-Alkoxy, C₁-C₄-Alkylcarbonyl, C₁-C₄-Alkoxycarbonyl, Halogen-C₁-C₄-alkyl, Halogen-C₁-C₄-alkoxy, C₁-C₄-Alkylthio und C₁-C₄-Alkylsulfonyl substituiertes Phenyl, oder R² und R³ mit dem sie tragenden Kohlenstoffatom einen gesättigten 5- oder 6-atomigen Ring bildend,
und
- X: Amino, Hydroxy, C₁-C₆-Alkoxy,C₃-C₈-Cycloalkoxy, C₁-C₆-Alkylamino, Di-(C₁-C₆-alkyl)amino, C₁-C₆-Alkyloxy-C₁-C₆-alkyloxy, Tri-(C₁-C₆-alkyl)-silyloxy und Tri-(C₁-C₆)-silylmethyloxy
bedeuten.

Die Bezeichnung "Halogen" umfaßt Fluor, Chlor, Brom und Jod.

Ein gegebenenfalls substituierter organischer Rest bedeutet gegebenenfalls substituiertes Phenyl, unsubstituiertes oder durch ein oder mehrere gleiche oder verschiedene Halogenatome substituiertes Alkyl, Alkenyl, Alkinyl, Cycloalkyl, Alkoxyalkyl, Alkylcarbonyl, Alkylaminocarbonyl, Dialkylaminocarbonyl mit jeweils bis zu 10 Kohlenstoffatomen im jeweiligen Kohlenwasserstoffrest.

Unter "C₁-C₆-Alkyl" ist ein unverzweigter oder verzweigter Kohlenwasserstoffrest mit ein, zwei, drei, vier, fünf oder sechs Kohlenstoffatomen zu verstehen, zum Beispiel Methyl, Ethyl, Propyl, 1-Methylethyl, Butyl, 1-Methylpropyl, 2-Methylpropyl, 1,1-Dimethylethyl, Pentyl, 2-Methylbutyl, 1,1-Dimethylpropyl und Hexyl. Unter zusammengesetzten Bezeichnungen, wie "C₁-C₆-Alkoxy", "C₁-C₆-Alkylamino" und "Tri-(C₁-C₆)-silyloxy", ist eine Alkoxy, Alkylamino- beziehungsweise Silyloxygruppe zu verstehen, deren Alkylreste sinngemäß die unter dem Ausdruck "C₁-C₆-Alkyl" angegebene Bedeutung haben. "Di-(C₁-C₆-alkyl)-amino" bedeutet, daß die beiden Alkylreste gleich oder verschieden sein können.

C₃-C₈-Cycloalkyl steht für Cyclopropyloxy, Cyclobutyloxy, Cyclopentyloxy, Cyclohexyloxy, Cycloheptyloxy und Cyclooctyloxy.

Die Bezeichnungen "Alkenyl" und "Alkinyl" bedeuten, daß die Kohlenstoffkette verzweigt oder unverzweigt sein kann und mindestens eine Mehrfachbindung beinhaltet, wobei sich diese an beliebiger Position des betreffenden ungesättigten Restes befinden kann.

Gegebenenfalls substituiertes Phenyl bedeutet, daß ein oder mehrere Wasserstoffatome des Phenylrestes durch gleiche oder verschiedene Substituenten aus der Gruppe Amino, Carboxyl, Cyano, Formyl, Halogen, Hydroxy, Nitro, C₁-C₄-Alkyl, C₁-C₄-Alkoxy, C₁-C₄-Alkylcarbonyl, C₁-C₄-Alkoxycarbonyl, Halogen-C₁-C₄alkyl, Halogen-C₁-C₄-alkoxy, C₁-C₄-Alkylthio und C₁-C₄-Alkylsulfonyl ersetzt sind.

"Halogenalkoxy" und "Halogenalkyl" bedeuten, daß ein oder mehrere Wasserstoffatome durch die entsprechende Anzahl gleicher oder verschiedener Halogenatome substituiert sind.

Ein 5- oder 6-atomiger Ring steht für einen carbo- oder heterocyclischen Rest, in dem bis zu 2 Ringatome aus der Gruppe Stickstoff, Sauerstoff und Schwefel stammen können, wobei dieser Ring gesättigt oder teilgesättigt und gegebenenfalls durch 1 oder 2 Methylgruppen substituiert sein kann, zum Beispiel Cyclopentyl, Cyclohexyl, Cyclohexenyl und 3-Oxacyclopentyl.

Im erfindungsgemäßen Verfahren enthält die eine Phase (organische Phase) zu Beginn der Reaktion das Hydrazon (im folgenden auch Komponente I genannt), das Olefin (im folgenden auch Komponente II genannt) und gegebenenfalls organische Lösungsmittel und die zweite Phase enthält Wasser (wäßrige Phase). Je nach Löslichkeit ist das sterisch gehinderte Amin in der organischen und/oder in der wäßrigen Phase gelöst, während die weitere Base in der Regel nahezu vollständig in der wäßrigen Phase gelöst oder suspendiert ist. Mit fortschreitender Reaktion sinkt der Anteil der Komponenten I und II in der organischen Phase, während der Anteil der Verbindung der allgemeinen Formel III zunimmt. Ebenso wie mit fortschreitender Reaktion der Anteil der Base in der wäßrigen Phase sinkt, steigt in dieser der Anteil an Salz, das aus dem während der Reaktion gebildeten Halogenwasserstoff und der Base entsteht.

Als Lösungsmittel eignen sich grundsätzlich alle organischen Lösungsmittel, die unter den Reaktionsbedingungen des erfindungsgemäßen Verfahrens inert sind, d.h. keine unerwünschten Reaktionen eingehen. Besonders geeignet sind Lösungsmittel aus der Gruppe aliphatische, cycloaliphatische, ungesättigte aliphatische und aromatische Kohlenwasserstoffe, die jeweils gegebenenfalls chloriert sein können, wie Hexan, Ligroin, Petrolether, Cyclohexan, Benzol, Toluol, Xylol, Chlorbenzol, Methylenchlorid, Chloroform, Kohlenstofftetrachlorid, Ethylenchlorid und Trichlorethylen, aliphatische Ketone, wie Acetone, Methylethylketon, Methylisopropylketon und Methylisobutylketon, Carbonsäureester, wie Ethylacetat und Amylacetat, Carbonsäureamide, wie N-Methylpyrrolidon, Dimethylformamid und Dimethylacetamid, Carbonsäurenitrile wie Acetonitril und Propionitril, Sulfoxide, wie Dimethylsulfoxid sowie Sulfone, wie Sulfolan. Selbstverständlich kann auch ein Gemisch der genannten Lösungsmittel verwendet werden. Ebenso kann das Verfahren auch ohne organisches Lösungsmittel durchgeführt werden.

Das Molverhältnis zwischen Komponente II und Komponente I kann in einem weiten Bereich gewählt werden und liegt üblicherweise bei 1 bis 20, bevorzugt 1 bis 10, besonders bevorzugt 1 bis 5. Wird das erfindungsgemäße Verfahren ohne Lösungsmittel durchgeführt, so sollte zweckmäßigerweise der Anteil von Komponente II höher gewählt werden. Das Molverhältnis zwischen Komponente II und Komponente I liegt dann vorzugsweise bei 2 bis 20, besonders bevorzugt 2 bis 10. Ein weiterer Vorteil des erfindungsgemäßen Verfahrens liegt auch darin, daß in diesem Fall nach Reaktionsende die überschüssige Komponente II zusammen mit sterisch gehindertem Amin abdestilliert und wieder für einen neuen Reaktionsansatz verwendet werden kann.

Für das erfindungsgemäße Verfahren eignen sich alle sterisch gehinderten Amine, die in der Lage sind, den bei der Reaktion freiwerdenden Halogenwasserstoff zu binden. Besonders geeignet sind Amine aus der Gruppe Dialkylamine, wie Diisopropylamin, Trialkylamine, wie Triethylamin und Tributylamin, Dialkylbenzylamine, wie N,N-Dimethylbenzylamin, Alkyldibenzylamine sowie aromatische Amine, wie Pyridine. Sie können sowohl in katalytischen wie auch in mindestens stöchiometrischen Mengen eingesetzt werden. Üblicherweise werden sie in einem Molverhältnis von 0,001 bis 2, bevorzugt von 0,01 bis 2, besonders bevorzugt von 0,01 bis 0,1, bezogen auf Komponente I verwendet.

Wird das stöchiometrisch gehinderte Amin in einer katalytischen, d.h. unterstöchiometrischen Menge eingesetzt, so wird zweckmäßigerweise zusätzlich eine weitere Base verwendet. Hierzu eignen sich solche Basen, die ebenfalls in der Lage sind, den bei der Reaktion freiwerdenden Halogenwasserstoff zu binden. Besonders geeignet sind Basen aus der Gruppe Alkali- und Erdalkalicarbonate, wie Lithium-, Natrium-, Kalium-, Magnesium- und Calciumcarbonat, Alkali- und Erdalkalihydrogencarbonate, wie Natrium-, Kalium-, Magnesium- und Calciumhydrogencarbonat, Alkali- und Erdalkalihydroxide, wie Lithium-, Natrium-, Kalium-, Magnesium- und Calciumhydroxid, Alkaliacetate, wie Natriumacetat, sowie Alkalialkoholate, wie Natriummethylat, Natriumethylat, Kalium-tert.-butylat. Üblicherweise werden sie in einem Molverhältnis von 0 bis 200, bevorzugt von 0 bis 100, besonders bevorzugt von 10 bis 100, bezogen auf sterisch gehindertes Amin, verwendet. Zur Erzielung einer optimalen Ausbeute sollte die Gesamtmenge an Base, d.h. die Menge an sterisch gehinderten Amin und weiterer Base, mindestens so groß gewählt werden, daß der bei der Reaktion entstehende Halogenwasserstoff vollständig durch die Base gebunden wird.

Die zur Durchführung des erfindungsgemäßen Verfahrens notwendige Wassermenge kann ebenfalls in einem weiten Bereich gewählt werden. Sie sollte so groß sein, daß sie während der Reaktion entstehenden Salze aus Halogenwasserstoff, sterisch gehindertem Amin und weiterer Base aufzunehmen vermag. Üblicherweise liegt das Molverhältnis zwischen Wasser und Komponente II in einem Bereich von 2 bis 100, bevorzugt von 2 bis 50. Dabei bezieht sich das angegebene Molverhältnis auf die Gesamtmenge des eingesetzten Wassers, d.h. auf die zu Beginn der Reaktion vorgelegte Wassermenge und die gegebenenfalls der im Verlauf der Reaktion zugetropfte - weitere Base enthaltende - Wassermenge.

Ein besonderer Vorteil des erfindungsgemäßen Verfahrens besteht auch darin, daß das relativ teure sterisch gehinderte Amin in lediglich katalytischen Mengen eingesetzt werden kann und durch die weitere verhältnismäßig preiswerte Base laufend regeneriert wird.

Selbstverständlich können Zusatzstoffe, wie Polyethylenglykole, quartäre Ammoniumsalze und Kronenether zugegeben werden, um beispielsweise die Löslichkeit der Basen zu erhöhen. Diese Zusatzstoffe sind dem Fachmann bekannt.

Die Herstellung der Komponenten I ist beispielsweise in WO 91/07874 beschrieben. Die Komponenten II sind in der Regel kommerziell erhältlich oder können nach dem Fachmann bekannten Methoden hergestellt werden.

Das erfindungsgemäße Verfahren wird üblicherweise so durchgeführt, daß die Komponenten I und II, sterisch gehindertes Amin, Wasser und gegebenenfalls organisches Lösungsmittel in ein Reaktionsgefäß gefüllt werden. Falls das sterisch gehinderte Amin nicht katalytisch sonders mindestens stöchiometrisch eingesetzt wird, erfolgt keine Zugabe weitere Base, und die Reaktionsmischung wird auf die erforderliche Reaktionstemperatur gebracht und bei dieser Temperatur gerührt. Die erforderliche Reaktionstemperatur hängt im wesentlichen von der Reaktivität der verwendeten Komponenten I und II ab. Sie liegt üblicherweise zwischen 0 und 150°C, bevorzugt zwischen 20 und 120°C. Das Verfahren kann bei Normaldruck oder verminderten Druck durchgeführt werden. Wird ein Lösungsmittel verwendet, so ist darauf zu achten, daß dessen Siedepunkt mindestens so hoch liegt wie die notwendigen Reaktionstemperatur. Weiterhin ist insbesonders bei phasenvermittelnden Lösungsmitteln, wie Dimethylformamid, Aceton und Methanol darauf zu achten, daß das Zweiphasen-System der Reaktionsmischung erhalten bleibt. Falls das sterisch gehinderte Amin katalytisch eingesetzt werden soll, wird eine weitere Base, die zweckmäßigerweise in einem geeigneten Lösungsmittel gelöst sein kann, bei der erforderlichen Reaktionstemperaturen der Reaktionsmischung in der Weise zugeführt, daß der pH-Wert der wäßrigen Phase der Reaktionsmischung in einem Bereich zwischen 6 und 9 liegt. Die Wahl des geeigneten Lösungsmittel richtet sich im wesentlichen nach der Art der eingesetzten Base. So kann beispielsweise für Basen wie Kaliumcarbonat und Natriumhydroxid Wasser verwendet werden, während für Alkoholate zweckmäßigerweise der dieser Base zugrundeliegende Alkohol verwendet wird, beispielsweise Methanol im Falle von Natriummethanolat. Die Kontrolle des pH-Wertes kann beispielsweise kontinuierlich mittels eines in die Reaktionsmischung tauchenden pH-Meters oder diskontinuierlich durch in kurzen Zeitabschnitten durchzuführenden Probennahmen erfolgen.

Die Reaktion, die etwa zwischen 2 und 48 Stunden dauern und beispielsweise dünnschichtchromatographisch verfolgt werden kann, wird vorzugsweise bis zum vollständigen Umsatz der Komponente I durchgeführt. Die Aufarbeitung der Reaktionsmischung kann nach allgemein bekannten Methoden, wie Destillation, Extraktion und/oder Filtration erfolgen. Die Aufarbeitungsmethode richtet sich nach den Eigenschaften der Reaktionsmischung. In der Regel kann die Reaktionsmischung zunächst von allen leichtflüchtigen Komponenten, wie Lösungsmittel, Wasser, sterisch gehindertem Amin und überschüssiger Komponente II durch Destillation befreit werden. Ein Vorteil des erfindungsgemäßen Verfahrens liegt darin, daß dieses Amin und Komponente II enthaltende Destillat in einem weiteren Reaktionsansatz verwendet werden kann. Zur weiteren Reinigung kann das Rohprodukt mit einem Lösungsmittel aus dem Destillationsrückstand extrahiert werden und nach Abziehen des Lösungsmittels chromatographisch, destillativ oder durch Kristallisation gereinigt werden.

Die Verbindungen der allgemeinen Formel I werden in der Regel nach dem erfindungsgemäßen Verfahren in höherer Ausbeute und Reinheit gewonnen als nach dem Stand der Technik. Zudem liegt der Gehalt an toxikologisch bedenklichem Hydrazon der allgemeinen Formel I deutlich niedriger. Weiterhin ist ein geringerer Materialeinsatz an Amin notwendig.

Das nachfolgende Vergleichsbeispiel demonstriert die Vorteile des erfindungsgemäßen Verfahrens gegenüber dem Stand der Technik.

### Beispiel

### Herstellung von 1-(2,4-Dichlorphenyl)-5-methyl-2-pyrazolin-3,5-dicarbonsäurediethylester (IIIa)

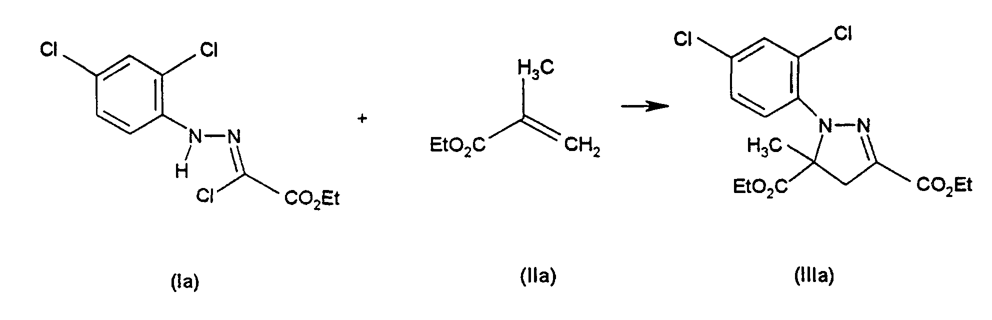

598,2 g (2 Mol) Ethyl-2-chlor-(2,4-dichlorphenylhydrazono)carboxylat (la), 456 g (4 Mol) Ethylmethacrylat (IIa) und 10,1 g (0,1 Mol) Triethylamin werden mit 100 ml Wasser in einem Rührkolben vorgelegt. Bei einer Temperatur von 60 bis 65°C wird innerhalb von 2 Stunden eine Lösung von 195 g (1,95 Mol) Kaliumhydrogencarbonat in 600 ml Wasser so zugetropft, daß der pH-wert der wäßrigen Phase der Reaktionsmischung nicht über 8 steigt. Nach beendetem Zutropfen wird noch 20 min bei der oben angegebenen Temperatur gerührt. Das überschüssige Ethylmethacrylat wird mit Wasser und Triethylamin im Vakuum abdestilliert. Der Reaktionsrückstand wird mit Toluol extrahiert. Das so erhaltene Toluolextrakt wird vom Lösungsmittel befreit und anschließend im Feinvakuum destilliert. Man erhält 754,2 g (98 % der Theorie) 1-(2,4-Dichlorphenyl)-5-methyl-2-pyrazolin-3,5-dicarbonsäure-diethylester (IIIa) mit einem Schmelzpunkt von 42-44°C und einer Reinheit von 97 % (Bestimmung per HPLC).

Die folgende Tabelle zeigt die relevanten Reaktionsparameter und Analysenwerte des erfindungsgemäßen Verfahrens (Versuch A) im Vergleich zum Stand der Technik (Versuch B).

**Tabelle**

| Versuch | Stoffmengen [Moläquivalente] | | | | Ausbeute [%] | Reinheit [%] | Gehalt an la [ppm] |
|---|---|---|---|---|---|---|---|
| | Komponente | | Base | Wasser | | | |
| | Ia | IIa | | | | | |
| A | 1 | 2 | 0,05 Net₃ + 1,0 KHCO₃ | 50 | 98 | 97 ¹⁾ | < 5 ³⁾ |
| B | 1 | 4 | 1,5 NEt₃ | 0 | 85 | 88²⁾ | 900 |

| | | | | | | | |
|---|---|---|---|---|---|---|---|
| ¹⁾ Feststoff mit einem Schmelzpunkt von 42 bis 44°C | | | | | | | |
| ²⁾ Öl mit einem Brechungsindex n_{D}²⁰ = 1.5651 | | | | | | | |
| ³⁾ unterhalb der Nachweisgrenze | | | | | | | |

## Patentansprüche

1. Verfahren zur Herstellung von 1-Phenylpyrazolin-3-carbonsäure-Derivaten der allgemeinen Formel III durch basenkatalysierte Reaktion von Hydrazonen der allgemeinen Formel I mit Olefinen der allgemeinen Formel II 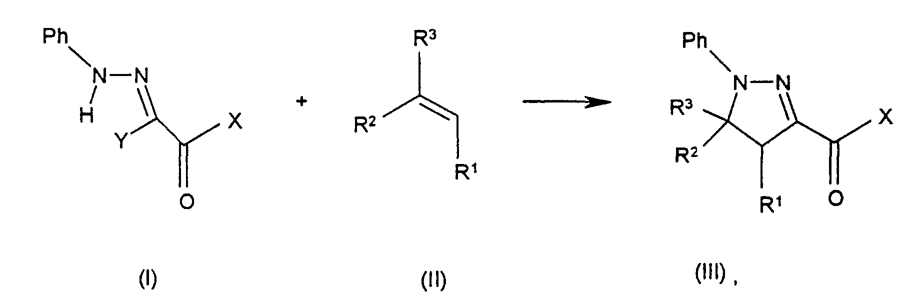 worin
Ph gegebenenfalls substituiertes Phenyl,
R¹ Wasserstoff oder Alkyl,
R² und R³ unabhängig voneinander Wasserstoff, Halogen, Cyano, einen gegebenenfalls substituierten organischen Rest, oder R² und R³ mit dem sie tragenden Kohlenstoffatom einen gesättigten oder teilgesättigten 5- oder 6-atomigen Ring bildend,
X Amino, Hydroxy, Alkoxy, Cycloalkoxy, Alkylamino, Dialkylamino, Alkyloxyalkyloxy, Trialkylsilyloxy oder Trialkylsilylmethyloxy und
Y Chlor oder Brom
bedeuten,
**dadurch gekennzeichnet, daß** die Reaktion in einem Zweiphasen-System in Anwesenheit eines sterisch gehinderten Amins und gegebenenfalls einer weiteren Base durchgeführt wird.

2. Verfahren nach Anspruch 1, **dadurch gekennzeichnet, daß** die eine Phase Hydrazon, Olefin und gegebenenfalls mindestens ein organisches Lösungsmittel und die andere Phase Wasser enthält.

3. Verfahren nach Anspruch 1 oder 2, **dadurch gekennzeichnet, daß** das sterisch gehinderte Amin aus der Gruppe Dialkylamine, Trialkylamine, Dialkylbenzylamine, Alkyldibenzylamine, aromatische Amine und die weitere Base aus der Gruppe Alkali- und Erdalkalicarbonate, Alkali- und Erdalkalihydrogencarbonate, Alkali- und Erdalkalihydroxide, Alkaliacetate und Alkalialkoholate stammt.

4. Verfahren nach einem der Ansprüche 1 bis 3, **dadurch gekennzeichnet, daß** die Reaktion ohne Lösungsmittel durchgeführt wird.

5. Verfahren nach einem der Ansprüche 1 bis 4, **dadurch gekennzeichnet, daß** die weitere Base so zudosiert wird, daß der pH-Wert der wäßrigen Phase des Zweiphasen-Systems zu Beginn, während und am Ende der Reaktion in einem Bereich zwischen 6 und 9 liegt.

6. Verfahren nach einem der Ansprüche 1 bis 5, **dadurch gekennzeichnet, daß** die Reaktion bei Normaldruck oder verminderten Druck und bei einer Temperatur zwischen 25 und 120°C durchgeführt wird.

7. Verfahren nach einem der Ansprüche 1 bis 6, **dadurch gekennzeichnet, daß**
R² und R³ unabhängig voneinander Wasserstoff, Halogen, Cyano, C₁-C₆-Alkyl, C₃-C₆-Cycloalkyl, C₂-C₆-Alkenyl, C₂-C₆-Alkinyl, Halogen-C₁-C₆-alkyl, C₁-C₆-Alkoxy-C₁-C₆-alkyl, C₁-C₆-Alkylcarbonyl, C₁-C₆-Alkoxycarbonyl, C₁-C₆-Alkylaminocarbonyl, Di-(C₁-C₆-alkyl)-aminocarbonyl, unsubstituiertes oder durch ein oder mehrere gleiche oder verschiedene Reste aus der Gruppe Amino, Carboxyl, Cyano, Formyl, Halogen, Hydroxy, Nitro, C₁-C₄-Alkyl, C₁-C₄-Alkoxy, C₁-C₄-Alkylcarbonyl, C₁-C₄-Alkoxycarbonyl, Halogen-C₁-C₄-alkyl, Halogen-C₁-C₄-alkoxy, C₁-C₄-Alkylthio und C₁-C₄-Alkylsulfonyl substituiertes Phenyl, oder R² und R³ mit dem sie tragenden Kohlenstoffatom einen gesättigten 5- oder 6-atomigen Ring bildend,
und
X Amino, Hydroxy, C₁-C₆-Alkoxy,C₃-C₈-Cycloalkoxy, C₁-C₆-Alkylamino, Di-(C₁-C₆-alkyl)amino, C₁-C₆-Alkyloxy-C₁-C₆-alkyloxy, Tri-(C₁-C₆-alkyl)-silyloxy und Tri-(C₁-C₆)-silylmethyloxy
bedeuten.

8. Verfahren nach einem der Ansprüche 1 bis 7, **dadurch gekennzeichnet, daß** das Molverhältnis zwischen sterisch gehindertem Amin und Hydrazon 0,01 bis 2, und das Molverhältnis zwischen weiterer Base und sterisch gehindertem Amin 0 bis 100 beträgt.

9. Verfahren nach Anspruch 8, **dadurch gekennzeichnet, daß** das Molverhältnis zwischen sterisch gehindertem Amin und Hydrazon 0,01 bis 0,1 und daß das Molverhältnis zwischen weiterer Base und sterisch gehindertem Amin 10 bis 100 beträgt.

10. Verfahren nach einem der Ansprüche 1 bis 9, **dadurch gekennzeichnet, daß** das Molverhältnis zwischen Olefin und Hydrazon 1 bis 10 beträgt.

11. Verfahren nach Anspruch 10, **dadurch gekennzeichnet, daß** das Molverhältnis zwischen Olefin und Hydrazon 1 bis 5 beträgt.

12. Verfahren nach einem der Ansprüche 1 bis 11, **dadurch gekennzeichnet, daß** das Molverhältnis zwischen Wasser und Olefin 2 bis 50 beträgt.

## Claims

1. A process for the preparation of 1-phenylpyrazoline-3-carboxylic acid derivatives of the formula III by means of base-catalyzed reaction of hydrazones of the formula I with olefins of the formula II 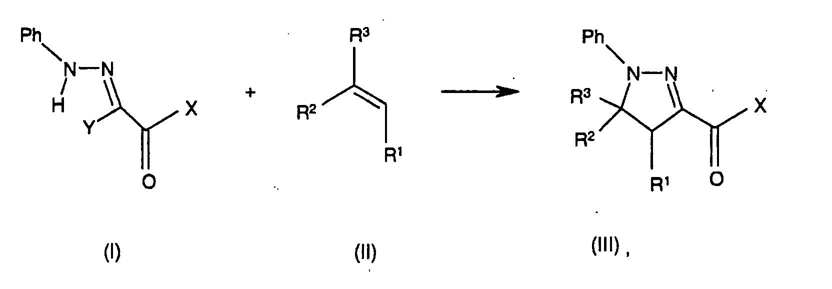 where
Ph is optionally substituted phenyl,
R¹ is hydrogen or alkyl,
R² and R³ independently of one another are hydrogen, halogen, cyano, an optionally substituted organic radical, or R² and R³ together with the carbon atom to which they are attached forming a saturated or partially saturated ring having 5 or 6 atoms,
X is amino, hydroxyl, alkoxy, cycloalkoxy, alkylamino, dialkylamino, alkyloxyalkyloxy, trialkylsilyloxy or trialkylsilylmethyloxy and
Y is chlorine or bromine,
which comprises carrying out the reaction in a two-phase system in the presence of a sterically hindered amine and, if appropriate, of a further base.

2. The process as claimed in claim 1, wherein the one phase comprises hydrazone, olefin and, if appropriate, at least one organic solvent and the other phase comprises water.

3. The process as claimed in claim 1 or 2, wherein the sterically hindered amine is selected from the group consisting of dialkylamines, trialkylamines, dialkylbenzylamines, alkyldibenzylamines, aromatic amines, and the further base is selected from the group consisting of alkali metal carbonates, alkaline earth metal carbonates, alkali metal hydrogen carbonates, alkaline earth metal hydrogen carbonates, alkali metal hydroxides, alkaline earth metal hydroxides, alkali metal acetates and alkali metal alkoxides.

4. The process as claimed in any of claims 1 to 3, wherein the reaction is carried out without solvent.

5. The process as claimed in any of claims 1 to 4, wherein the further base is metered in in such a way that the pH of the aqueous phase of the two-phase system is within a range of between 6 and 9 at the beginning, during and at the end of the reaction.

6. The process as claimed in any of claims 1 to 5, wherein the reaction is carried out under atmospheric pressure or reduced pressure and at a temperature of between 25 and 120°C.

7. The process as claimed in any of claims 1 to 6, wherein
R² and R³ independently of one another are hydrogen, halogen, cyano, C₁-C₆-alkyl, C₃-C₆-cycloalkyl, C₂-C₆-alkenyl, C₂-C₆-alkynyl, halo-C₁-C₆-alkyl, C₁-C₆-alkoxy-C₁-C₆-alkyl, C₁-C₆-alkylcarbonyl, C₁-C₆-alkoxycarbonyl, C₁-C₆-alkylaminocarbonyl, di-(C₁-C₆-alkyl)-aminocarbonyl, phenyl which is unsubstituted or substituted by one or more identical or different radicals selected from the group consisting of amino, carboxyl, cyano, formyl, halogen, hydroxyl, nitro, C₁-C₄-alkyl, C₁-C₄-alkoxy, C₁-C₄-alkylcarbonyl, C₁-C₄-alkoxycarbonyl, halo-C₁-C₄-alkyl, halo-C₁-C₄-alkoxy, C₁-C₄-alkylthio and C₁-C₄-alkylsulfonyl, or R² and R³ together with the carbon atom to which they are attached forming a saturated ring having 5 or 6 atoms,
and
X is amino, hydroxyl, C₁-C₆-alkoxy, C₃-C₈-cycloalkoxy, C₁-C₆-alkylamino, di-(C₁-C₆-alkyl)amino, C₁-C₆-alkyloxy-C₁-C₆-alkyloxy, tri-(C₁-C₆-alkyl)silyloxy and tri-(C₁-C₆)silylmethyloxy.

8. The process as claimed in any of claims 1 to 7, wherein the molar ratio between sterically hindered amine and hydrazone is 0.01 to 2 and the molar ratio between further base and sterically hindered amine is 0 to 100.

9. The process as claimed in claim 8, wherein the molar ratio between sterically hindered amine and hydrazone is 0.01 to 0.1 and the molar ratio between further base and sterically hindered amine is 10 to 100.

10. The process as claimed in any of claims 1 to 9, wherein the molar ratio between olefin and hydrazone is 1 to 10.

11. The process as claimed in claim 10, wherein the molar ratio between olefin and hydrazone is 1 to 5.

12. The process as claimed in any of claims 1 to 11, wherein the molar ratio between water and olefin is 2 to 50.

## Revendications

1. Procédé pour la préparation de dérivés d'acide 1-phénylpyrazolin-3-carboxylique de formule générale III par réaction catalysée par une base d'hydrazones de formule générale I avec des oléfines de formule générale II 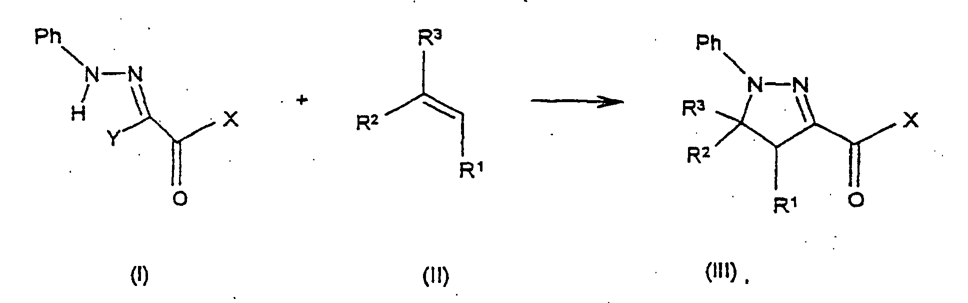 où
Ph représente un groupe phényle éventuellement substitué,
R¹ représente un atome d'hydrogène ou un groupe alkyle,
R² et R³ représentent indépendamment l'un de l'autre un atome d'hydrogène, un atome d'halogène, un groupe cyano, un reste organique éventuellement substitué, ou R² et R³ représentent avec l'atome de carbone les portant un cycle saturé ou en partie saturé de 5 ou 6 chaînons,
X représente un groupe amino, hydroxy, alkoxy, cycloalkoxy, alkylamino, dialkylamino, alkyloxyalkyloxy, trialkylsilyloxy ou trialkylsilylméthyloxy et
Y représente un atome de chlore ou de brome,
**caractérisé en ce que** la réaction est réalisée dans un système biphasique en présence d'une amine à encombrement stérique et éventuellement d'une autre base.

2. Procédé selon la revendication 1, **caractérisé en ce que** une phase contient l'hydrazone, une oléfine et éventuellement au moins un solvant organique et l'autre phase contient l'eau.

3. Procédé selon la revendication 1 ou 2, **caractérisé en ce que** l'amine encombrée stériquement est prise dans le groupe comprenant des dialkylamines, trialkylamines, dialkylbenzylamines, alkyldibenzylamines, amines aromatiques et l'autre base est prise dans le groupe comprenant des carbonates alcalins et alcalino-terreux, bicarbonates alcalins et alcalino-terreux, hydroxydes alcalins et alcalino-terreux, acétates alcalins et alcoolates alcalins.

4. Procédé selon l'une des revendications 1 à 3, **caractérisé en ce que** la réaction est réalisée sans solvant.

5. Procédé selon l'une des revendications 1 à 4, **caractérisé en ce que** l'autre base est ajoutée progressivement de façon telle que la valeur du pH de la phase aqueuse du système biphasique au début, pendant et à la fin de la réaction se trouve dans un intervalle entre 6 et 9.

6. Procédé selon l'une des revendications 1 à 5, **caractérisé en ce que** la réaction est réalisée sous pression normale ou pression réduite et à une température entre 25 et 120°C.

7. Procédé selon l'une des revendications 1 à 6, **caractérisé en ce que**
R² et R³ représentent indépendamment l'un de l'autre un atome d'hydrogène, un atome d'halogène, un groupe cyano, alkyle en C₁-C₆, cycloalkyle en C₃-C₆, alcényle en C₂-C₆, alcynyle en C₂-C₆, halogénoalkyle en C₁-C₆, alkoxy en C₁-C₆-alkyle en C₁-C₆, (alkyl en C₁-C₆)carbonyle, (alkoxy en C₁-C₆)carbonyle, (alkyl en C₁-C₆)aminocarbonyle, di-(alkyl en C₁-C₆)-aminocarbonyle, phényle non substitué ou substitué par un ou plusieurs restes identiques ou différents de l'ensemble amino, carboxyle, cyano, formyle, halogène, hydroxy, nitro, alkyle en C₁-C₄, alkoxy en C₁-C₄, (alkyl en C₁-C₄)carbonyle, (alkoxy en C₁-C₄)carbonyle, halogénoalkyle en C₁-C₄, halogènoalkoxy en C₁-C₄, (alkyl en C₁-C₄)thio et (alkyl en C₁-C₄)sulfonyle, ou R² et R³ représentent avec l'atome de carbone les portant un cycle saturé de 5 ou 6 chaînons,
et
X représente un groupe amino, hydroxy, alkoxy en C₁-C₆, cycloalkoxy en C₃-C₈, alkylamino en C₁-C₆, di-(alkyl en C₁-C₆)amino, (alkyloxy en C₁-C₆)-(alkyloxy en C₁-C₆), tri-(alkyl en C₁-C₆)silyloxy ou tri-(alkyl en C₁-C₆)silylméthyloxy.

8. Procédé selon l'une des revendications 1 à 7, **caractérisé en ce que** le rapport molaire entre l'amine à encombrement stérique et l'hydrazone s'élève à 0,01 à 2 et le rapport molaire entre l'autre base et l'amine à encombrement stérique s'élève à 0 à 100.

9. Procédé selon la revendication 8, **caractérisé en ce que** le rapport molaire entre l'amine à encombrement stérique et l'hydrazone s'élève à 0,01 à 0,1 et que le rapport molaire entre l'autre base et l'amine à encombrement stérique s'élève à 10 à 100.

10. Procédé selon l'une des revendications 1 à 9, **caractérisé en ce que** le rapport molaire entre l'oléfine et l'hydrazone s'élève à 1 à 10.

11. Procédé selon la revendication 10, **caractérisé en ce que** le rapport molaire entre l'oléfine et l'hydrazone s'élève à 1 à 5.

12. Procédé selon l'une des revendications 1 à 11, **caractérisé en ce que** le rapport molaire entre l'eau et l'oléfine s'élève à 2 à 50.
